# EUROPÄISCHE PATENTSCHRIFT

(11) **EP 0 940 081 B1**
(45) Veröffentlichungstag und Bekanntmachung des Hinweises auf die Patenterteilung: **26.06.2002**
(21) Anmeldenummer: 99103771.4
(22) Anmeldetag: 26.02.1999
(51) Int. Cl.: A01N 47/42

(54) **Wirkstoffkombinationen und Desinfektionsmittel, Konservierungsmittel und Antiseptika dieselben enthaltend**
Combinations of active agents and disinfectants, preservatives and antiseptics containing same
Associations d'agents actifs et désinfectants, préservatifs et antiseptiques les contenant

(30) Priorität: 04.03.1998 DE 19808962
(43) Veröffentlichungstag der Anmeldung: 08.09.1999
(73) Patentinhaber: Bode Chemie GmbH & Co., D-22525 Hamburg (DE)
(72) Erfinder: Jülich, Wolf-Dieter, Dr., 17489 Greifswald (DE); Seibt, Horst, Dr., 10407 Berlin (DE); Pietsch, Hanns, Dr., 20148 Hamburg (DE); Kramer, Axel, Prof. Dr. med., 17487 Greifswald (DE); Ohme, Roland, Dr., 12527 Berlin (DE); Ballschuh, Detlef, Dr., 12524 Berlin (DE); Knieler, Roland, Dr., 22529 Greifswald (DE); Adrian, Vera, Dr., 17493 Greifswald (DE)
(74) Vertreter: Dinné, Erlend

(56) Entgegenhaltungen:
- N. WEIGAND ET AL.: "Vergleigende Untersuchung der antimikrobiellen Wirksamkeit von S-Alkylthiuroniumhalogeniden." ZENTRALBLATT FÜR BAKTERIOLOGIE, PARASITENKUNDE, INFEKTIONSKRANKHEITEN UND HYGIENE, ERSTE ABTEILUNG ORIGINALE-REIHE A, Bd. 237, Nr. 1, 1977, Seiten 118-123, XP002106274

## Beschreibung

Die vorliegende Erfindung betrifft Wirkstoffkombinationen, die mindestens ein Alkylthiouronium- und/oder α, ω-Alkylendithiouroniumsalz und mindestens ein Peroxid enthalten, sowie Desinfektionsmittel, Konservierungsmittel und Antiseptika dieselben enthaltend.

Als Mittel zur Desinfektion, Konservierung und Antiseptik sind eine Vielzahl mikrobizid wirksamer chemischer Substanzen bzw. Gemische dieser Substanzen an sich bekannt. Mikrobizide Substanzen sind im allgemeinen gegen das übliche Spektrum von Keimen, wie beispielsweise grampositive Bakterien, gramnegative Bakterien, Mykobakterien, Hefen, Pilze, Viren und dergleichen, mehr oder weniger wirksam, so daß man üblicherweise eine ausreichende Desinfektion, Konservierung oder Antiseptik durch geeignete Wirkstoffkombinationen erzielen kann.

Der Stand der Technik kennt zur Desinfektion, Konservierung und Antiseptik eine Reihe von Wirkstoffen, insbesondere Aldehyde, wie beispielsweise Formaldehyd oder Glutaraldehyd, quaternäre Ammoniumverbindungen und langkettige Amine, Phenole oder Alkohole. Allerdings weisen die genannten Verbindungen einige Nachteile auf:

Aldehyde fixieren Reste von Blut und Eiweiß durch chemische Reaktion an den zu desinfizierenden Gegenständen, so daß diese nach der Desinfektion schwer zu reinigen sind. Außerdem haben sie ein vergleichsweise hohes allergenes Potential, so daß Anwendungen auf Haut und Händen nur in geringen Konzentrationen möglich sind oder aber in Kombination mit weiteren Wirkstoffen in Betracht kommen, um die erforderliche Unterschreitung der Sensibilisierungsschwelle einhalten zu können. Höhere Konzenauch aus diesem Grund die Konzentration durch Kombination mit weiteren Wirkstoffen verringert. Die Anwendungsbereiche von Aldehyden werden ferner durch die umstrittene karzinogene Potenz sowie die Mutagenität dieser Stoffklasse weiter eingeschränkt.

Quaternäre Ammoniumverbindungen und langkettige Amine werden häufig in der Flächendesinfektion und zur manuellen Instrumentendesinfektion sowie in geringem Umfang auch in der Händeantiseptik verwendet. Im Vergleich zu den Aldehyden ist der Geruch dieser Verbindungen deutlich weniger unangenehm. Eine chemische Reaktion mit Eiweißen erfolgt nicht, jedoch kommt es zu einer physikalischen Fällung von Eiweißen, die zum Teil durch geschickte Kombination mit Tensiden kompensiert werden kann. Für die maschinelle Instrumentendesinfektion sind die quaternären Ammoniumverbindungen nicht geeignet, weil es infolge der Turbulenzen in der Reinigungsmaschine zu einer starken, unerwünschten Schaumbildung kommt. Bei der Flächendesinfektion zeigen quaternäre Ammoniumverbindungen eine starke Tendenz, auf den Oberflächen "aufzuziehen", d. h. es bilden sich Schichten dieser Verbindungen auf den Oberflächen aus. Ein weiterer entscheidender Nachteil ist das eingeengte Wirkungsspektrum quaternärer Ammoniumverbindungen, da diese weder sporozid noch gegen unbehüllte Viren wirken.

Phenole sind vor allem wegen ihres Geruches, ihrer geringen Wirksamkeit gegen den Poliovirus, ihrer zum Teil schlechten Abbaubarkeit, ihrer hohen Lipidlöslichkeit verbunden mit einer starken Penetration durch die Haut sowie toxischer und mutagener Risiken in nahezu allen Anwendungsbereichen für Desinfektionsmittel auf dem Rückzug.

Auch die Verwendung von Peroxiden in Desinfektonsmitteln, Konservierungsmitteln und Antiseptika ist an sich bekannt. Als Peroxide können beispielsweise organische Persäuren oder Alkylperoxide eingesetzt werden. Während diese Mittel ursprünglich zumeist als Bleich- und Desinfektionsmittel in Waschmitteln eingesetzt wurden, nimmt beispielsweise ihre Verwendung in Flächendesinfektionsmitteln mittlerweile zu. Nachteilig sind allerdings ihre geringe Haltbarkeit in Lösung, eine starke Konzentrationsabnahme in Gegenwart organischer Substanzen und ein langsamer Wirkungseintritt. Ein weiterer Nachteil von Peroxiden ist auch ihr eingeengtes Wirkungsspektrum, insbesondere gegen Pilze. Speziell bei Wasserstoffperoxid kommt als weiterer Nachteil eine hohe Cytotoxizität hinzu.

Ein speziell eingesetztes Peroxid ist Magnesiummonoperphthalat. Dieses ist gegenüber unterschiedlichen Erregern unterschiedlich wirksam, d. h. seine antimikrobielle Wirkung zeigt eine Konzentrationsabhängigkeit. So wird beispielsweise Candida albicans, der Soor-Pilz, erst bei höheren Konzentrationen bzw. nach längeren Einwirkungszeiten abgetötet.

Die hohe antimikrobielle Wirksamkeit von Dodecylthiouroniumchlorid ist an sich bekannt (*Zbl Bakt. Hyg., 1 Orig. A 237, 117-123, 1977*). Allerdings ist diese Verbindung - ebenso wie andere Thiouroniumsalze - in wäßriger Lösung labil, neigt z.B. in Gegenwart von Metallsalzen zum Ausflocken, und ist daher nur schwer in Formulierungen einzuarbeiten.

Als Thiouroniumsalze werden im folgenden Alkylthiouronium- und/oder α, ω-Alkylendithiouroniumsalze bezeichnet. Alkylthiouroniumsalze zeichnen sich durch folgende chemische Struktur aus 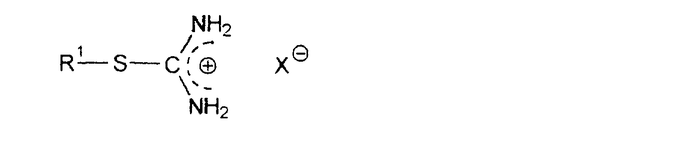 wobei R¹ gewählt wird aus der Gruppe der verzweigten und unverzweigten Alkylgruppen mit 1 bis 20 Kohlenstoffatomen und X⁻ ein beliebiges Anion darstellt, vorzugsweise aber ein Halogenid-lon.

α, ω-Alkylendithiouroniumsalze sind durch die folgende Struktur gekennzeichnet 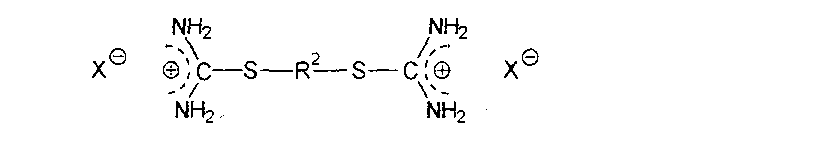 wobei R² gewählt wird aus der Gruppe der verzweigten und unverzweigten Alkylgruppen mit 1 bis 20 Kohlenstoffatomen und X⁻ ein beliebiges Anion darstellt, vorzugsweise aber ein Halogenid-lon.

Aufgabe der Erfindung war es, einen Wirkstoff bzw. eine Wirkstoffkombination zu finden, der bzw. die eine hohe bakterizide und fungizide Wirkung zeigt, in wäßriger Lösung stabil sowie gut verträglich ist.

Es war indes überraschend und für den Fachmann nicht vorauszusehen, daß Wirkstoffkombinationen, die dadurch gekennzeichnet sind, daß sie mindestens ein Alkylthiouronium- und/oder α, ω-Alkylendithiouroniumsalz und mindestens ein Peroxid enthalten, den geschilderten Nachteilen des Standes der Technik Abhilfe schaffen würden.

Wirkstoffkombinationen im Sinne der vorliegenden Erfindung sind hervorragend bakterizid, fungizid und überraschenderweise auch sporozid, obwohl die einzelnen Komponenten nicht sporozid wirksam sind. Sie sind geeignete Wirkstoffe in Desinfektionsmitteln, Konservierungsmitteln und Antiseptika, sie eignen sich zur Desinfektion von Flächen und chirurgischen Instrumenten und zur Konservierung, insbesondere zur Konservierung von kosmetischen oder dermatologischen Zubereitungen.

Eine besonders vorteilhafte Ausführungsform, die ihrerseits erfinderisch ist, besteht aus der Kombination von mindestens einem Thiouroniumsalz mit Wasserstoffperoxid und Harnstoff. Es ist gegebenenfalls von Vorteil, das Wasserstoffperoxid und den Harnstoff in Form von Percarbamid (Harnstoffperoxohydrat H₂N-CO-NH₂ • H₂O₂) einzusetzen. Ebenfalls vorteilhaft ist es, im Handel erhältliches, mit Stärke tablettiertes Percarbamid zu verwenden. Die Menge an Harnstoff wird vorzugsweise aus dem Bereich von 0 bis 75 Gewichtsanteilen gewählt, bezogen auf das Teilgewicht der Mischung von Wasserstoffperoxid und Harnstoff. Besonders bevorzugt besteht die Mischung von Wasserstoffperoxid und Harnstoff zu 30 Gewichtsanteilen aus Wasserstoffperoxid und zu 70 Gewichtsanteilen aus Harnstoff.

Die Wirkstoffkombination von mindestens einem Thiouroniumsalz mit Harnstoff und Wasserstoffperoxid zeigt eine hervorragende Stabilität in wäßriger Lösung und eine sehr gute Hautverträglichkeit. Die cytotoxische Wirkung auf humane Amnionepitelzellen (FL-Zellen) ist im Vergleich zu den Einzelkomponenten deutlich herabgesetzt. Noch deutlicher zeigt sich die geringe toxische Wirkung dieser Wirkstoffkombination in Perfusionskulturen mit FL-Zellen. Die erfindungsgemäße Dreistoffkombination ist wegen ihrer Stabilität und Verträglichkeit insbesondere als Konservierungsmittel geeignet, z. B. zur Konservierung von kosmetischen Zubereitungen wie beispielsweise Waschlotionen. Cremes oder Hautlotionen, wo sie vorzugsweise in Mengen von 0,1 bis 5,0 Gew.-% eingearbeitet wird, bezogen auf das Gesamtgewicht dieser Zubereitungen.

Bei der Kombination von Thiouroniumsalzen mit anionischen Wirkstoffen kann ein deutlicher Synergismus in der Wirkung gegenüber verschiedenen bakteriellen und pilzlichen Erregern nachgewiesen werden. Beispielsweise zeigen erfindungsgemäße Kombinationen von Magnesiummonoperphthalat und mindestens einem Alkylthiouronium- und/oder α, ω-Alkylendithiouroniumsalz keine Wirkungslücke gegenüber Candida-Spezies. Dies war insbesondere deswegen nicht zu erwarten, weil sich schwerlösliche Salze, wie beispielsweise das Decylthiouroniumperphthalat, bilden können und davon auszugehen war, daß durch Ausfällungen die Wirksamkeit des Desinfektionsmittels herabgesetzt wird.

Sofern als Peroxide im Sinne der vorliegenden Erfindung Salze von organischen Persäuren bzw. anionische Peroxide verwendet werden, ist es vorteilhaft, das Verhältnis von Peroxid zu Thiouroniumsalz in der Wirkstoffkombination aus dem Bereich 999 : 1 bis 10 : 1, insbesondere wie 700 : 1 bis 25 : 1 zu wählen.

Als Peroxide im Sinne der vorliegenden Erfindung können beispielsweise Wasserstoffperoxid (H₂O₂), organische Persäuren, Alkylperoxide oder Alkylhydroperoxide eingesetzt werden. Die organischen Persäuren können aliphatische oder aromatische Verbindungen sein und als solche oder in Form ihrer Alkali-, Erdalkali- oder Ammoniumsalze vorliegen. Beispiele für aliphatische Persäuren sind Perfettsäuren, z. B. Perfettsäuren mit 3 bis 12 Kohlenstoffatomen. Beispiele für aromatische Persäuren sind Perbenzoesäure, Perphthalsäure und Perbenzoesäurederivate wie z. B. p-Methoxyperbenzoesäure oder Benzoylperoxid. Besonders bevorzugt ist Magnesiummonoperphthalat, vor allem wegen seiner außergewöhnlich breiten Materialverträglichkeit.

Wirkstoffkombinationen im Sinne der vorliegenden Erfindung sowie Desinfektionsmittel, Konservierungsmittel und Antiseptika dieselben enthaltend, in denen Magnesiummonoperphthalat das Peroxid ist, eignen sich insbesondere zur schonenden Desinfektion von hochsensiblen Gerätschaften sowie zur Flächendesinfektion in patientennahen Bereichen.

Weitere vorteilhafte Peroxide im Sinne der vorliegenden Erfindung sind Alkylperoxide (R―O―O―R') und/oder Alkylhydroperoxide (R"―O―OH), wobei R, R' und R" unabhängig voneinander gewählt werden aus der Gruppe der Alkylgruppen mit gerader oder verzweigter Kohlenstoffkette mit einer Kettenlänge von 2 bis 8 Kohlenstoffatomen, wie beispielsweise t-Butylhydroperoxid [(CH₃)₃C―O―OH] sowie Perborate, Peroxodisulfate, Persulfate, Percarbonate und dergleichen mehr, beispielsweise Kaliumperoxodisulfat, Kaliumpersulfat und Natriumpercarbonat.

Vorzugsweise wird das Verhältnis von Peroxid zu Thiouroniumsalz in der erfindungsgemäßen Wirkstoffkombination gewählt aus dem Bereich 999 : 1 bis 1: 999. insbesondere wie 555 : 1 bis 1 : 555.

Es ist bevorzugt, den Gehalt an einem oder mehreren Alkylthiouronium- und/oder α, ω-Alkylendithiouroniumsalz(en) in dem Desinfektionsmittel, Konservierungsmittel oder Antiseptikum zwischen 0,05 und 30 Gew.-%, bezogen auf das Gesamtgewicht der Zubereitung, zu wählen.

Die erfindungsgemäßen Wirkstoffkombinationen sowie Desinfektionsmittel, Konservierungsmittel und Antiseptika dieselben enthaltend können in Form von festen Konzentraten, z. B. in (Brause-)Tabletten-, Pulver-, Pellet- oder Granulatform, vorliegen und beispielsweise in Dosierbeutel abgepackt werden, so daß eine Gebrauchslösung jeweils frisch angesetzt werden kann. Sie können aber auch in Form von Lösungen oder Suspensionen in nicht-wäßrigen Lösungsmitteln vorliegen. Eine Besonderheit sind Wirkstoffkombinationen von mindestens einem Thiouroniumsalz, Wasserstoffperoxid und Harnstoff, welche auch in wäßrigen Lösungen, Emulsionen oder Cremes über lange Zeit stabil sind.

Bevorzugt sind Desinfektionsmittel, Konservierungsmittel und Antiseptika, die neben den erfindungsgemäßen Wirkstoffen weitere antimikrobielle Wirkstoffe enthalten. Alkylthiouronium- und/oder α, ω-Alkylendithiouroniumsalze sind geeignete Kombinationspartner für nahezu alle bekannten Wirkstoffe.

Erfindungsgemäße Desinfektionsmittel, Konservierungsmittel und Antiseptika können vorzugsweise auch Tenside enthalten, wie sie üblicherweise Verwendung finden, beispielsweise Sulfobetaine, Alkylsulfonate, Alkylbenzolsulfonate und/oder nichtionische Tenside auf Basis von Ethylenoxid und/oder Butylenoxid.

Ferner können die erfindungsgemäßen Desinfektionsmittel, Konservierungsmittel und Antiseptika vorteilhaft zusätzlich übliche Korrosionsinhibitoren, wie beispielsweise Benzotriazol, enthalten, insbesondere falls sie bei ihrer Anwendung mit metallischen Oberflächen, insbesondere mit nicht veredelten Stählen oder Werkstoffen aus Buntmetallegierungen, in Berührung kommen können.

Zusätzlich zu den vorstehend genannten Komponenten können die erfindungsgemäßen Desinfektionsmittel, Konservierungsmittel und Antiseptika für derartige Zubereitungen übliche Farbstoffe, Duftstoffe und/oder andere übliche Hilfsstoffe, wie beispielsweise Dickungs-, Granulierungs-, Tablettierungs- oder Dispergierhilfsmittel, enthalten. Diese tragen in der Mehrzahl der Fälle nicht zur desinfizierenden, konservierenden oder antiseptischen Wirkung bei, sondern dienen der Handhabbar- bzw. Lagerbarkeit sowie ästhetischen Zwecken. Es ist jedoch auch möglich, solche Komponenten zu verwenden. die eine (konservierende, pflegende usw.) Wirkung entfalten und dabei gleichzeitig für eine bestimmte Farbe und/oder einen angenehmen Duft sorgen.

Die jeweils einzusetzenden Mengen an derartigen Trägerstoffen und Parfüm können in Abhängigkeit von der Art des jeweiligen Produktes vom Fachmann durch einfaches Ausprobieren leicht ermittelt werden.

Die folgenden Beispiele dienen dazu, die Erfindungen zu beschreiben, selbstverständlich ohne daß beabsichtigt ist, die Erfindungen auf diese Beispiele zu beschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

### Beispiel 1:

### Bakteriostatische und fungistatische Wirkung von Kombinationen von Magnesiummonoperphthalat (MMPP) mit Alkylthiouroniumsalzen

**Methodik der Prüfung auf bakteriostatische und fungistatische Wirksamkeit im Röhrchenverdünnungstest**: In sterile Reagenzröhrchen werden je 5 ml der Verdünnungen der Prüfsubstanzen in Wasser standardisierter Härte mit 5 ml doppelt konzentrierter Caseinpepton-Sojamehl-Pepton-Lösung (CSL) vermischt. Zur Bestimmung der bakteriostatischen Wirkung werden die Röhrchen durch Zugabe von 0,1 ml einer 1 : 10 mit CSL verdünnten, 24 h bei 37 °C bebrüteten CSL-Kultur beimpft. Zur Prüfung der fungistatischen Wirkung werden je 0,1 ml einer unverdünnten, 72 h bei 37 °C bebrüteten CSL-Kultur von Candida albicans verwendet. Die Auswertung erfolgt nach einer Bebrütung von 72 h bei 37 °C.
Es erfolgte außerdem eine Nachbeimpfung von CSA-Platten durch Ausstreichen der "72 h-Testlösungen", um das Wachstum der Testkeime zu beurteilen. Folgende Testkeime wurden eingesetzt:
- Sa:: Staphylococcus aureus ATCC 6538
- Ef:: Enterococcus faecium ATCC 6057
- Ec:: Escherichia coli ATCC 11229
- Pm:: Proteus mirabilis ATCC 14153
- Pa:: Pseudomonas aeruginosa ATCC 15442
- Ca:: Candida albicans ATCC 10231

**Ergebnisse:** Bei 3 Testkeimen - Candida albicans, Enterococcus faecium und Proteus mirabilis - ist das Gemisch wirksamer als MMPP allein (Tab.) Die Summe der Hemmkonzentrationen für den resistentesten Keim Candida albicans im Gemisch q₁₊₂ (gemessen in Einheiten der Hemmkonzentration der Einzelsubstanz) beträgt 0,71. Da der Sollwert für Additivität unterschritten wird, liegt nach Poser et al. (*Z. med. Labortechnik* ***5*** *(1964) 293-300)* ein Synergismus vor. In Tabelle 1 bedeuten + Wachstum und - kein Wachstum.

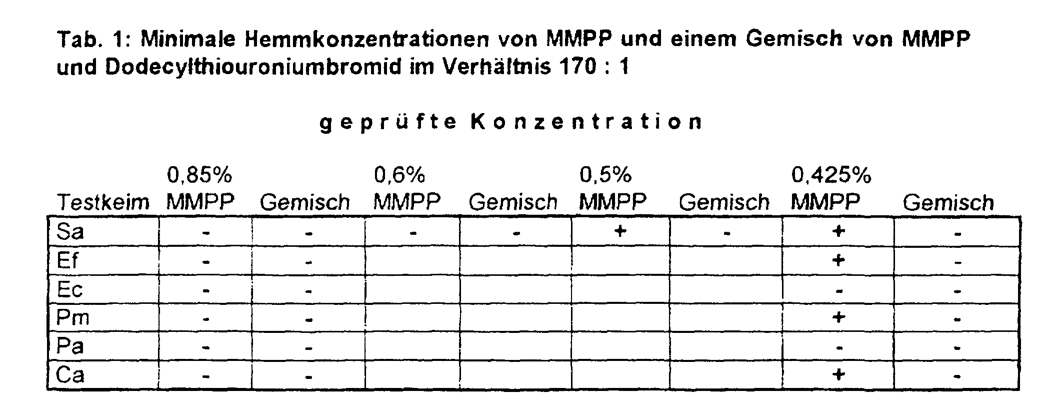

### Beispiel 2:

### Prüfung auf mikrobizide Wirksamkeit

**Methodik**: Untersucht wurde ein Gemisch G 1 von 99,4 % MMPP und 0,6 % Decylthiouroniumbromid und ein Gemisch G 2 von 99,7 % MMPP und 0,3 % Decylthiouroniumbromid im Vergleich zu MMPP als Monowirkstoff entsprechend der Richtlinie für die Prüfung und Bewertung chemischer Desinfektionsverfahren der Deutschen Gesellschaft für Hygiene und Mikrobiologie.
Testkeim: Candida albicans ATCC 10231
Enthemmer: Tween, Lecithin, Cystein (TLC)

### Ergebnisse:

Die Wirkungslücke von MMPP gegen Candida albicans wird aufgehoben.

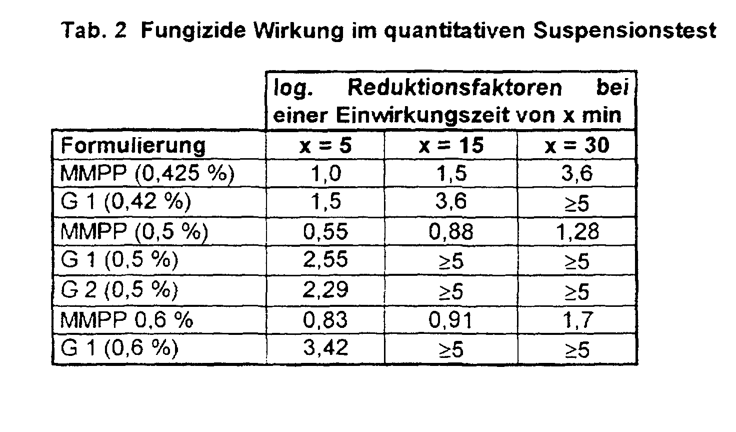

### Beispiel 3:

### Prüfung auf Kombinationseffekte der Kombination von Thioroniumsalzen und MMPP im Blockierung-Aktievierung-Test (BAT) nach Kramer et al. (1991)

**Methodik**: Das Testprinzip besteht darin, daß Dodecylthiouroniumbromid (DDTB) in 3 keine Wachstumshemmung bewirkenden Verdünnungen separat in Caseinpepton-Sojamehlpepton-Agar inkorporiert wird und MMPP in 4 verschiedenen Konzentrationen in ausgestanzte Agarlöcher einpipettiert wird. Die Agarlöcher haben 10 mm Durchmesser und werden am Boden mit einem Agartropfen abgedichtet. Entstehen auf den Platten mit ungehemmtem Keimwachstum Hemmhöfe bzw. nehmen die Hemmhofdurchmesser im Vergleich zur Kontrollplatte ohne Wirkstoffzusatz im Agar zu, liegt eine Wirkungsverstärkung vor.
Testkeim: Candida albicans ATCC 10231

### Ergebnisse:

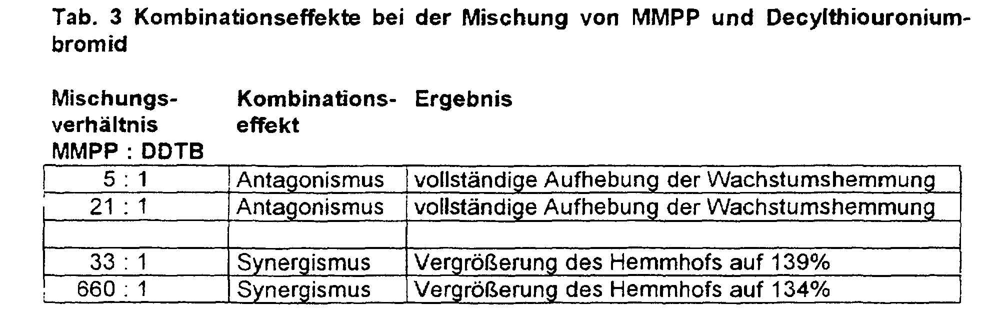

Bei einem zu hohem Anteil von Dodecylthiouroniumbromid kommt es erwartungsgemäß zu antagonistischen Effekten, da sich schwerlösliche Salze bilden können. In einem weiten Konzentrationsbereich (33:1 bis 660:1) kommt es dagegen überraschend zu synergistischen Effekten.

### Beispiel 4:

### Untersuchungen der Kombination von Decylthiouroniumbromid mit aktivierten Perboraten im Agardiffusionstest

**Methodik:** Decylthiouroniumbromid wurde in abgestuften Konzentrationen im Agar inkorporiert. In Stanzlöcher wurde eine Natriumperboratlösung ohne und mit Aktivierung pipettiert. Die Platten wurden mit Staphylococcus aureus beimpft und 24 h bebrütet. Der entstehende Hemmhof wurde gemessen.

**Ergebnisse**: Die bakteriostatische Wirkung der Perborate ist bei Zimmertemperatur gering. Die bakteriostatische Wirkung der Perborate wird durch Einsatz eines Aktivators (an polymeres Material fixierte Silberionen, Gehalt 8,0 x 10⁻³) aktiviert. Der Aktivator hat in der eingesetzten Konzentration keine bakteriostatische Eigenwirkung. Bei der Kombination von aktivierten Perboraten mit Alkylthiouroniumsalzen wird eine beträchtliche Wirkungssteigung beobachtet.

**Tab. 4**

| **Ergebnisse des Agardiffusionstestes** | | | | | |
|---|---|---|---|---|---|
| **Konzentration an Decylthiouroniumbromid im Agar** | **Aktivator** | **Hemmhofgröße (mm) bei Perboratkonzentration im Stanzloch** | | | |
| | | **2%** | **1%** | **0,5%** | **0,25%** |
| - | nein | 13 | 11 | 0 | 0 |
| - | ja | 19 | 15 | 13 | 0 |
| 0,0009% | ja | | | 19 | |
| 0,0019% | ja | | | 24 | |
| Kontrolle: Aktivator allein | | 0 | 0 | 0 | 0 |

### Beispiel 5:

### Antimikrobielle Wirksamkeit von Kombinationen von Wasserstoffperoxid, Alkylthiouroniumsalzen und Harnstoff

**Methodik**: Percarbamid und Dodecylthiouroniumchlorid werden unter städigem Durchmischen bis zur vollständigen Homogenisierung erwärmt. Die Prüfung der Kombination im Vergleich zu den entsprechenden Monowirkstoffen auf bakteriostatische Wirksamkeit erfolgte im Agarinkorporationstest. Die zu untersuchenden Substanzen wurden in Wasser standardisierter Härte gelöst. Die Konzentration der Testsubstanzen im Nähragar wurde in einer geometrischen Reihe auf der Basis 2 abgestuft. Zum Beimpfen der Testplatten wurden die im Beispiel 1 genannten Testkeime 6 h in Caseinpepton-Sojamehlpepton-Lösung bebrütet und so in eine logarithmische Wachstumsphase gebracht. Danach wurden die Agarplatten sternförmig sektorenweise mit jeweils einer "abgestrichenen Öse" beimpft. Der Sektor von Proteus mirabilis wurde durch Gräben abgetrennt, um das Schwärmen zu verhindern. Nach 24 h Bebrütung bei 36 °C erfolgte die Bewertung des Bakterienwachstums unter Vergleich mit substanzfreien Kontrollplatten nach folgendem Schema:

| | |
|---|---|
| ++++ | ungehemmtes Wachstum |
| +++ | geringe Wachstumshemmung (maximal ¾ des Wachstums der Kontrollen) |
| ++ | mäßige Wachstumshemmung (maximal ½ des Wachstums der Kontrollen) |
| + | starke Wachstumshemmung (maximal ¼ des Wachstums der Kontrollen) Das sog. "verminderte Wachstum" (v. W.) umfaßt die Bereiche + bis +++ |
| - | absolute Hemmung (a. H.) |

In den Tabellen 5, 6 und 7 bedeuten ⊕⊕ ungehemmtes Wachstum und ⊕ vermindertes Wachstum.

### Ergebnisse:

**Tab. 5**

| **Bakteriostatische Wirksamkeit der Kombination mit 2,5 % Dodecylthiouroniumchlorid und 16 % Wasserstoffperoxid in Harnstoff (ad 100 %)** | | | | | | | | | | |
|---|---|---|---|---|---|---|---|---|---|---|
| **Testkeim** | **Konzentration der Wirkstoffkombination in Wasser (%)** | | | | | | | | | |
| | **0,8** | **0,4** | **0,2** | **0,1** | **0,05** | **0,025** | **0,012** | **0,006** | **0,003** | **0,0015** |
| Sa⁽¹⁾ | - | - | - | - | - | ⊕ | ⊕ | ⊕⊕ | ⊕⊕ | ⊕⊕ |
| Ef⁽²⁾ | - | - | - | - | - | - | - | ⊕ | ⊕⊕ | ⊕⊕ |
| Ec⁽³⁾ | - | - | - | - | ⊕ | ⊕ | ⊕⊕ | ⊕⊕ | ⊕⊕ | ⊕⊕ |
| Pm⁽⁴⁾ | - | - | - | - | - | ⊕ | ⊕ | ⊕⊕ | ⊕⊕ | ⊕⊕ |
| Pa⁽⁵⁾ | - | - | - | - | ⊕ | ⊕⊕ | ⊕⊕ | ⊕⊕ | ⊕⊕ | ⊕⊕ |

**Tab. 6**

| **Bakteriostatische Wirksamkeit von zum Vergleich untersuchten Wasserstoffperoxid als Einzelsubstanz** | | | | | | | | | | |
|---|---|---|---|---|---|---|---|---|---|---|
| **Testkeim** | **Konzentration des Wirkstoffs in Wasser (%)** | | | | | | | | | |
| | **0,8** | **0,4** | **0,2** | **0,1** | **0,05** | **0,025** | **0,012** | **0,006** | **0,003** | **0,0015** |
| Sa⁽¹⁾ | - | - | - | - | - | - | - | ⊕⊕ | ⊕⊕ | ⊕⊕ |
| Ef⁽²⁾ | - | - | - | - | - | - | - | ⊕ | ⊕⊕ | ⊕⊕ |
| Ec⁽³⁾ | - | - | - | - | - | - | - | ⊕⊕ | ⊕⊕ | ⊕⊕ |
| Pm⁽⁴⁾ | - | - | - | - | - | - | - | ⊕⊕ | ⊕⊕ | ⊕⊕ |
| Pa⁽⁵⁾ | - | - | - | - | - | ⊕⊕ | ⊕⊕ | ⊕⊕ | ⊕⊕ | ⊕⊕ |

**Tab. 7**

| **Bakteriostatische Wirksamkeit von zum Vergleich untersuchten Dodecylthiouroniumchlorid als Einzelsubstanz** | | | | | | | | | | |
|---|---|---|---|---|---|---|---|---|---|---|
| **Testkeim** | **Konzentration des Wirkstoffs in Wasser (%)** | | | | | | | | | |
| | **0,8** | **0,4** | **0,2** | **0,1** | **0,05** | **0,025** | **0,012** | **0,006** | **0,003** | **0,0015** |
| Sa⁽¹⁾ | - | - | - | - | - | - | - | - | ⊕⊕ | ⊕⊕ |
| Ef⁽²⁾ | - | - | - | - | - | - | - | ⊕ | ⊕⊕ | ⊕⊕ |
| Ec⁽³⁾ | - | - | - | - | - | - | ⊕ | ⊕⊕ | ⊕⊕ | ⊕⊕ |
| Pm⁽⁴⁾ | - | - | - | - | - | ⊕ | ⊕ | ⊕⊕ | ⊕⊕ | ⊕⊕ |
| Pa⁽⁵⁾ | - | - | - | - | ⊕ | ⊕⊕ | ⊕⊕ | ⊕⊕ | ⊕⊕ | ⊕⊕ |

| | | | | | | | | | | |
|---|---|---|---|---|---|---|---|---|---|---|
| ⁽¹⁾ Staphylococcus aureus | | | | | | | | | | |
| ⁽²⁾ Enterococcus faecium | | | | | | | | | | |
| ⁽³⁾ Escherichia coli | | | | | | | | | | |
| ⁽⁴⁾ Proteus mirabilis | | | | | | | | | | |
| ⁽⁵⁾ Pseudomonas aeruginosa | | | | | | | | | | |

Der resistenteste Testkeim Pseudomonas aeruginosa wird durch 0,1%ige Lösungen des Dodecylthiouroniumchlorids bzw. durch 0,05 % Wasserstoffperoxid gehemmt. In der Kombination sind dafür nur 0,0025 % des Thiouroniumsalzes und 0,016 % Wasserstoffperoxid erforderlich. Die Prüfung auf Kombinationseffekte nach Poser et al. (*Z.med Labortechnik* ***5*** *(1964) 293-300*) ergibt q_{Kombination} = 0,016/0,05 + 0,0025/0,1 = 0,34 und beweist den Synergismus der antimikrobiellen Wirkung. Die übrigen Testkeime werden durch noch geringere Konzentrationen gehemmt.

### Beispiel 6:

### Nachweis der sporiziden Wirkung

**Methodik:** Kommerzielle Teststreifen mit 10⁶ Sporen von Bacillus subtilis var. niger ATCC 9372 (BAG-Bio Strips, Biologische Analysensystem GmbH Lich) wurden für 3 Tage in eine Lösung der Prüfsubstanz eingelegt. Danach wurden die Teststreifen in eine Nährlösung überführt und 7 Tage bebrütet.

**Ergebnisse**: Nach einer Einwirkungszeit von 3 Tagen einer 0,3 %igen Lösung der Kombination nach Beispiel 5 wird eine Reduktion der Sporen von 6 log-Stufen gefunden. Wird Wasserstoffperoxid als Monowirkstoff angewandt, ist zur Erreichung diese Effektes eine Konzentration von 0,6 % erforderlich.

### Beispiel 7:

### Herabsetzung der Zytotoxität von Peroxiden durch Formulierungen mit Alkylthiouroniumsalzen

**Methodik:** Die Zytotoxizität wird an der FL-Linie (menschliche Amnionzellen) geprüft. 2-3 Tage nach der Zelleinsaat werden die Zellen, nachdem sie als einschichtige Zellkultur (Monolayer) vorliegen, nach Medienwechsel für 24 h mit der Prüfsubstanz exponiert, danach abgelöst und die Zellzahl in % zur Kontrolle mit dem Zellzähler Universal 87001 (mölab, Hilden) ermittelt.

**Ergebnisse:** Durch die erfindungsgemäße Formulierung nach Beispiel 5 wird die zytotoxische Wirkung auf humane Amnionepithelzellen im Vergleich zu den Einzelkomponenten deutlich herabgesetzt. Schon 0,0015 %ige Lösungen von Wasserstoffperoxid (entspricht 3 % der minimalen Hemmkonzentration von Wasserstoffperoxid gegenüber Pseudomonas aeruginosa) bewirken als Monowirkstoff eine Herabsetzung der Zellzahl der Amnionepithelzellen auf 7,3 %. 0,005 %ige Lösungen des Dodecylthiouroniumchlorids (entspricht ebenfalls 3 % der minimalen Hemmkonzentration gegen Pseudomonas aeruginosa) setzen das Zellwachstum auf 70 % herab. Dagegen wird durch 0,001-0,005%ige Lösungen der Kombination nach Beispiel 5 (entspricht wiederum 1-5 % der minimalen Hemmkonzentration gegen Pseudomonas aeruginosa) das Zellwachstum sogar stimuliert, wobei der 1%igen Lösung mit einer Steigerung der Zellzahl auf 129,7 % die Signifikanzgrenze überschritten wird. Selbst bei einer 0,01%igen Lösung (entspricht 10 % der minimalen Hemmkonzentration) ist die Kombination deutlich weniger toxisch (Zellzahl 66,3). Wasserstoffperoxid führt bei einer Konzentration, die 10 % der minimalen Hemmkonzentration entspricht, zu einer vollständigen Inhibierung des Zellwachstums.

**Tab. 8**

| **Zytotxizität von Wasserstoffperoxid, Decylthiouroniumchlorid und der Formulierung nach Beispiel 5** | | |
|---|---|---|
| **Prüfsubstanz** | **Prüfkonzentration** | **Median der Zellzahl** |
| | % | (% der Kontrolle) |
| Decylthiouroniumbromid | 0,005 | 33,5 |
| | 0,0001 | 96,8 |
| Dodecylthiouroniumchlorid | 0,005 | 69,9 |
| | 0,0001 | 101,3 |
| Formulierung mit 2,5 % Dodecylthiouroniumchlorid und 16 % H₂O₂ in Harnstoff (ad 100 %) | 0,01 | 66,3 |
| | 0,005 | 117,5 |
| | 0,001 | 129,7 |
| Wasserstoffperoxid als Monowirkstoff | 0,0015 | 7,3 |
| | 0,001 | 17,9 |

### Beispiel 8:

### Einfluß auf den Glucosestoffwechsel in einer Perfusionszellkultur

**Methodik**: Die Untersuchungen wurden an einer Perfusionszellkultur mit humanen Amnionepithelzellen durchgeführt. Der Glucoseverbrauch wurde kontinuierlich von 1 bis 96 Stunden biosensorisch gemessen (Jülich et al., im Druck). Die Prüfsubstanzen wirkten von der 25. bis 48. Stunde ein.

**Ergebnisse:** Wasserstoffperoxid bewirkt eine signifikante, aber reversible Verminderung des Glucosestoffwechsels der FL-Zellen. Demgegenüber wird der Glucosestoffwechsel durch die erfindungsgemäße Kombination nach Beispiel 5 in einer bakteriostatisch gleich wirksamen Konzentration nicht beeinflußt.

## Patentansprüche

1. Wirkstoffkombination, **dadurch gekennzeichnet, daß** sie mindestens ein Alkylthiouronium- und/oder α, ω-Alkylendithiouroniumsalz und mindestens ein Peroxid enthält.

2. Wirkstoffkombination nach Anspruch 1. **dadurch gekennzeichnet, daß** sie zusätzlich Harnstoff enthält.

3. Wirkstoffkombination nach einem der Ansprüche 1 oder 2, **dadurch gekennzeichnet, daß** sie als Peroxid Wasserstoffperoxid, organische Persäuren, deren Salze, Alkylperoxide, Alkylhydroperoxide oder deren Gemische enthält.

4. Wirkstoffkombination nach Anspruch 1, **dadurch gekennzeichnet, daß** sie als Peroxid Magnesiummonoperphthalat enthält.

5. Desinfektionsmittel, Konservierungsmittel und/oder Antiseptikum **dadurch gekennzeichnet, daß** es eine Wirkstoffkombination nach einem der Ansprüche 1 oder 2 enthält.

6. Desinfektionsmittel, Konservierungsmittel und/oder Antiseptikum nach Anspruch 5, **dadurch gekennzeichnet, daß** zusätzlich Tenside und/oder Hilfs-, Zusatz- und/oder Wirkstoffe enthalten sind.

7. Desinfektionsmittel, Konservierungsmittel und/oder Antiseptikum nach Anspruch 5, **dadurch gekennzeichnet, daß** zusätzlich weitere mikrobizide Wirkstoffe enthalten sind.

8. Desinfektionsmittel, Konservierungsmittel und/oder Antiseptikum nach Anspruch 5, **dadurch gekennzeichnet, daß** der Gehalt an einem oder mehreren Alkylthiouronium- und/oder α, ω-Alkylendithiouroniumsalzen 0,05 bis 30 Gew.-%, bezogen auf das Gesamtgewicht der Zubereitung, beträgt.

9. Wirkstoffkombination nach einem der Ansprüche 1 oder 2 oder Desinfektionsmittel, Konservierungsmittel und/oder Antiseptikum nach Anspruch 5, **dadurch gekennzeichnet, daß** es in Form eines festen Konzentrates vorliegt, insbesondere in Form von Tabletten, Granulat. Brausetabletten, Pellets oder als Pulver.

10. Verwendung von Wirkstoffkombinationen nach Anspruch 1 als Flächendesinfektionsmittel, insbesondere für harte Oberflächen, oder als Instrumentendesinfektionsmittel.

11. Verwendung von Wirkstoffkombinationen nach Anspruch 2 als Konservierungsmittel, insbesondere als Konservierungsmittel in kosmetischen oder dermatologischen Zubereitungen.

## Claims

1. Active compound combination, **characterized in that** it contains at least one alkylthiouronium and/or α,ω-alkylenedithiouronium salt and at least one peroxide.

2. Active compound combination according to Claim 1, **characterized in that** it additionally contains urea.

3. Active compound combination according to one of Claims 1 or 2, **characterized in that**, as peroxide, it contains hydrogen peroxide, organic peracids, their salts, alkyl peroxides, alkyl hydroperoxides or their mixtures.

4. Active compound combination according to Claim 1, **characterized in that**, as peroxide, it contains magnesium monoperphthalate.

5. Disinfectant, preservative and/or antiseptic, **characterized in that** it contains an active compound combination according to one of Claims 1 or 2.

6. Disinfectant, preservative and/or antiseptic according to Claim 5, **characterized in that** surfactants and/or auxiliaries, additives and/or active compounds are additionally contained.

7. Disinfectant, preservative and/or antiseptic according to Claim 5, **characterized in that** further microbicidal active compounds are additionally contained.

8. Disinfectant, preservative and/or antiseptic according to Claim 5, **characterized in that** the content of one or more alkylthiouronium and/or α,ω-alkylenedithiouronium salts is 0.05 to 30% by weight, based on the total weight of the preparation.

9. Active compound combination according to one of Claims 1 or 2, or disinfectant, preservative and/or antiseptic according to Claim 5, **characterized in that** it is present in the form of a solid concentrate, in particular in the form of tablets, granules, effervescent tablets, pellets or as a powder.

10. Use of active compound combinations according to Claim 1 as surface disinfectants, in particular for hard surfaces, or as instrument disinfectants.

11. Use of active compound combinations according to Claim 2 as preservatives, in particular as preservatives in cosmetic or dermatological preparations.

## Revendications

1. Association de substances actives, **caractérisée en ce qu'**elle contient au moins un sel d'alkylthio-uronium et/ou d'α,ω-alkylènedithio-uronium et au moins un peroxyde.

2. Association de substances actives selon la revendication 1, **caractérisée en ce qu'**elle contient en outre de l'urée.

3. Association de substances actives selon la revendication 1 ou 2, **caractérisée en ce qu'**elle contient en tant que peroxyde du peroxyde d'hydrogène, des peracides organiques, leurs sels, des alkylperoxydes, des alkylhydroperoxydes ou des mélanges de ceux-ci.

4. Association de substances actives selon la revendication 1, **caractérisée en ce qu'**elle contient en tant que peroxyde du monoperphtalate de magnésium.

5. Désinfectant, conservateur et/ou antiseptique, **caractérisé en ce qu'**il contient une association de substances actives selon la revendication 1 ou 2.

6. Désinfectant, conservateur et/ou antiseptique selon la revendication 5, **caractérisé en ce qu'**il contient en outre des tensioactifs et/ou des adjuvants, additifs et/ou substances actives.

7. Désinfectant, conservateur et/ou antiseptique selon la revendication 5, **caractérisé en ce qu'**il contient en outre d'autres substances actives microbicides.

8. Désinfectant, conservateur et/ou antiseptique selon la revendication 5, **caractérisé en ce que** la teneur en un ou plusieurs sels d'alkylthio-uronium et/ou d'α,ω-alkylènedithio-uronium va de 0,05 à 30 % en poids, par rapport au poids total de la préparation.

9. Association de substances actives selon la revendication 1 ou 2, ou désinfectant, conservateur et/ou antiseptique selon la revendication 5, **caractérisés en ce qu'**ils se présentent sous forme d'un concentré solide, en particulier sous forme de comprimés, produit granulé, comprimés effervescents, granules ou sous forme de poudre.

10. Utilisation d'associations selon la revendication 1, en tant que désinfectants pour surfaces, en particulier pour surfaces dures, ou en tant que désinfectants pour instruments.

11. Utilisation d'associations de substances actives selon la revendication 2 en tant que conservateur, en particulier en tant que conservateur dans des préparations cosmétiques ou dermatologiques.
